(19) Europäisches Patentamt
European Patent Office
Office européen des brevets

(11) **EP 4 019 122 A1**

(12) **EUROPEAN PATENT APPLICATION**
published in accordance with Art. 153(4) EPC

(43) Date of publication:
**29.06.2022 Bulletin 2022/26**

(21) Application number: **19948982.4**

(22) Date of filing: **16.10.2019**

(51) International Patent Classification (IPC):
**B01F 3/08** (2006.01)          **A61K 9/10** (2006.01)
**A61K 31/10** (2006.01)         **A61K 31/337** (2006.01)
**B01D 9/02** (2006.01)          **B01J 13/00** (2006.01)

(52) Cooperative Patent Classification (CPC):
**A61K 9/10; A61K 31/10; A61K 31/337; B01D 9/02; B01F 23/40; B01J 13/00**

(86) International application number:
**PCT/JP2019/040709**

(87) International publication number:
**WO 2021/075003 (22.04.2021 Gazette 2021/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Nano Cube Japan Co., Ltd.**
**Kita-ku**
**Okayama-shi**
**Okayama 701-1221 (JP)**
• **CBC Co., Ltd.**
**Tokyo 104-0052 (JP)**

(72) Inventors:
• **NAKAZAKI Yoshiaki**
**Okayama-shi, Okayama 701-1221 (JP)**
• **OTOYAMA Takafumi**
**Okayama-shi, Okayama 701-1221 (JP)**
• **MIYAIRI Shinya**
**Tokyo 104-0052 (JP)**

(74) Representative: **Regimbeau**
**20, rue de Chazelles**
**75847 Paris Cedex 17 (FR)**

(54) **METHOD FOR MANUFACTURING DISPERSION OF ULTRAFINE PARTICLES OF POORLY SOLUBLE SUBSTANCE**

(57)     The present invention provides a method for manufacturing a dispersion in which a substance, which is poorly soluble in a dispersion medium, is dispersed with a particle size of a nano-order level without using a highly toxic organic solvent as the dispersion medium. More particularly, provided is a method for manufacturing a dispersion in which a substance, which is poorly soluble in a dispersion medium containing a surfactant, is dispersed in the dispersion medium with a particle size of a nano-order level, as a dispersoid, the method including: preparing a solution containing a good solvent and the poorly soluble substance and the surfactant dissolved in the good solvent; rapidly cooling the solution to a temperature at which the poorly soluble substance precipitates in the solution at a temperature lowering rate of 100 to 4,000°C/second to produce ultrafine particles with a particle size of a nano-order level formed of the poorly soluble substance in the good solvent; and (i) separating the good solvent from a mixed solution of the solution and the dispersion medium after mixing the solution and the dispersion medium, or (ii) mixing the dispersion medium to the solution after separating the good solvent from the solution.

EP 4 019 122 A1

FIG. 1

**EP 4 019 122 A1**

**Description**

TECHNICAL FIELD

The present invention relates to a method for manufacturing a dispersion of a poorly soluble substance, and more particularly to a method for manufacturing a dispersion in which a substance, which is poorly soluble in a dispersion medium containing a surfactant, is dispersed in the dispersion medium with a particle size of a nano-order level, as a dispersoid.

BACKGROUND ART

It is known to use an organic solvent showing a certain level or higher of toxicity in a pharmaceutical preparation containing a poorly soluble substance having low solubility in a dispersion medium such as water, from the viewpoint of the solubility. However, from the viewpoint of quality of life (QOL), it is preferable that an organic solvent showing the toxicity is not used. Non-Patent Document 1 also mentions that the residual amount of the solvent in pharmaceuticals is strictly regulated, and ideally, a solvent having low toxicity as possible should be used.
On the other hand, there is known a method in which particles of a substance to be dispersed is microparticulated in order to disperse a poorly soluble substance having low solubility in a dispersion medium such as water in the dispersion medium. Examples of the microparticulation method include a method of grinding by applying a physical force such as shearing, collision or friction using a grinder such as a beads mill or a jet mill, or a homogenizer. However, if the physical force is applied to forcibly reduce the particle size of particles, the poorly soluble substance may be denatured or decomposed by impact, heat or the like.
Therefore, there is required a method of microparticulating without applying a physical force.
Under such technical circumstances, it can be said that there is a demand for a technology in which a poorly soluble substance is stably dispersed in a dispersion medium without denaturing or decomposing the poorly soluble substance even when a highly toxic organic solvent is not used as the dispersion medium.

PRIOR ART DOCUMENT

[Non-Patent Document]

[Non-Patent Document 1] Guideline for Residual Solvents (PMSB/ELD Notification No. 307, March 30, 1998)

SUMMARY OF THE INVENTION

Problems to be Solved by the Invention

Therefore, an object of the present invention is to provide a method for manufacturing a dispersion, capable of stably dispersing a substance, which is poorly soluble in a dispersion medium (hereinafter also simply referred to as poorly soluble substance), in the dispersion medium without denaturing or decomposing a poorly soluble substance even when a highly toxic organic solvent is not used as the dispersion medium. Another object of the present invention is to provide a dispersion obtained by the above manufacturing method, or ultrafine particles with a particle size of a nano-order level which contain the poorly soluble substance.

Means for Solving the Problems

The present inventors have intensively studied so as to solve the abovementioned problems and found that ultrafine particles with a particle size of a nano-order level formed of a poorly soluble substance can be obtained by rapidly cooling a solution containing a good solvent and a poorly soluble substance and a surfactant dissolved in the good solvent at a specific temperature lowering rate. They also found that the ultrafine particles with a particle size of a nano-order level formed of a poorly soluble substance are stably dispersed in an aqueous dispersion medium, thereby completing the present invention.
The following inventions are included in the present invention.

(1) A method for manufacturing a dispersion in which a substance, which is poorly soluble in a dispersion medium containing a surfactant, is dispersed in the dispersion medium with a particle size of a nano-order level, as a dispersoid, the method including:

preparing a solution containing a good solvent and the poorly soluble substance and the surfactant dissolved in the good solvent;

rapidly cooling the solution to a temperature at which the poorly soluble substance precipitates in the solution at a temperature lowering rate of 100 to 4,000°C/second to produce ultrafine particles with a particle size of a nano-order level formed of the poorly soluble substance in the good solvent; and

(i) separating the good solvent from a mixed solution of the solution and the dispersion medium after mixing the solution and the dispersion medium, or (ii) mixing the dispersion medium to the solution after separating the good solvent from the solution.

(2) The method according to (1), wherein the solution temperature is lowered using a micro heat exchanger.

(3) The method according to (1) or (2), wherein the separation of the good solvent in (i) is performed by distilling off the good solvent from the mixed solution of the solution and the dispersion medium.

(4) The method according to (1) or (2), wherein the separation of the good solvent in (ii) is performed by drying the good solvent.

(5) The method according to any one of (1) to (4), wherein the poorly soluble substance is at least one selected from the group consisting of coenzyme Q10, docetaxel, paclitaxel, busulfan, azadirachtin and ascorbic acid.

(6) The method according to any one of (1) to (5), wherein the dispersion medium is at least one selected from the group consisting of water, ethanol, propylene glycol and 1-butanol.

(7) The method according to any one of (1) to (6), wherein the good solvent is at least one solvent selected from the group consisting of acetone, ethanol, 2-propanol, propylene glycol and water.

(8) The method according to any one of (1) to (7), wherein the average particle size of the ultrafine particles is 0.5 nm or more and 300 nm or less.

(9) A method for manufacturing a powder containing ultrafine particles of a poorly soluble substance from the dispersion obtained in any one of (1) to (8), the method including optionally adding an excipient to the dispersion, and removing the dispersion medium to obtain a powder containing ultrafine particles of a poorly soluble substance.

(10) Ultrafine particles with a particle size of a nano-order level formed of a poorly soluble substance, wherein the average particle size of the fine particles is 0.5 nm or more and 300 nm or less, and the coefficient of variation of the particle size is 5 or less.

Effects of the Invention

According to the present invention, a solution in which a poorly soluble substance is dissolved in a good solvent containing a surfactant is rapidly cooled to produce ultrafine particles of the poorly soluble substance, and then (i) the good solvent is separated from a mixed solution of the solution and the dispersion medium after mixing the solution and the dispersion medium, or (ii) the dispersion medium is mixed to the solution after separating the good solvent from the solution, whereby, it is possible to produce a dispersion in which the poorly soluble substance is dispersed with a particle size of a nano-order level, and the dispersion has a feature that it is stably dispersed. The ultrafine particles of the present invention are advantageous in that they can be stably dispersed in a non-toxic or low-toxic dispersion medium such as water or ethanol. Further, the dispersion of the present invention is advantageous in that it can be produced without using a highly toxic solvent. Therefore, it is advantageous in that the occurrence of side effects due to the highly toxic solvent can be reduced.

BRIEF DESCRIPTION OF THE DRAWINGS

FIG. 1 shows a flow chart of the manufacturing method according to the present invention.

Fig. 2 is a transmission electron microscope (TEM) image (120,000 times) of ultrafine particles of coenzyme Q10 obtained in Example 1.

Fig. 3 shows the particle size distribution of ultrafine particles of coenzyme Q10 obtained in Example 1.

Fig. 4A shows photographs of a dispersion of ultrafine particles of busulfan obtained in Example 2 or dispersions of particles of busulfan obtained in Reference Example 2-1 and Reference Example 2-2 after being left to stand for 2 hours.

Fig. 4B shows photograph of laser irradiation of a dispersion of ultrafine particles of busulfan obtained in Example 2 or dispersions of busulfan particles obtained in Reference Example 2-1 and Reference Example 2-2 after being left to stand for 2 hours.

Fig. 5A shows photographs of a dispersion of ultrafine particles of docetaxel obtained in Example 3 or dispersions of particles of docetaxel obtained in Reference Example 3-1 and Reference Example 3-2 after being left to stand for 2 hours.

Fig. 5B shows photographs of laser irradiation of a dispersion of docetaxel ultrafine particles obtained in Example 3 or dispersions of docetaxel particles obtained in Reference Examples 3-1 and 3-2 after being left to stand for 2 hours.

DETAILED DESCRIPTION OF THE INVENTION

One of features of the method for manufacturing a dispersion of a poorly soluble substance according to the present invention includes rapidly cooling a solution in which the poorly soluble substance and the surfactant are dissolved in a good solvent to produce ultrafine particles of the poorly soluble substance in the good solvent; and (i) separating the good solvent from a mixed solution of the solution and the dispersion medium after mixing the solution and the dispersion medium, or (ii) mixing the dispersion medium to the solution after separating the good solvent from the solution.

<Substance which is poorly soluble in Dispersion Medium>

The substance which is poorly soluble in the dispersion medium in the present invention means a substance which is either insoluble or hardly soluble in the dispersion medium, and includes substances corresponding to "slightly soluble", "very slightly soluble" and "hardly soluble" mentioned in The Japanese Pharmacopoeia 17th edition. The poorly soluble substance in the present invention has, for example, a solubility in a dispersion medium of about 10 mg/mL or less, preferably 5 mg/mL or less, more preferably 0.5 mg/mL or less, still more preferably 0.3 mg/mL or less, yet more preferably 0.1 mg/mL or less, and further preferably 0.01 mg/mL or less, at a handling temperature of a usual pharmaceutical compound, for example, 20±5°C.

Examples of the poorly soluble substance in the present invention includes, but are not particularly limited, to those to be used, or to be used in future in pesticides, pharmaceuticals (including veterinary drugs), quasi-drugs, cosmetics, foods, food additives or supplements. Examples of the poorly soluble substance include, but are not particularly limited, to benzoquinone-based compounds such as coenzyme Q10, and derivatives thereof; taxane-based compounds such as docetaxel and paclitaxel, and derivatives thereof; limonoids such as azadirachtin; flavonoids such as rutin; alkylsulfonate-based compounds such as busulfan; water-soluble vitamins such as ascorbic acid; polyphenol-based compounds such as curcumin, and derivatives thereof; fat-soluble vitamins such as ascorbyl palmitate and tocopherol; sphingolipids such as ceramide NP and ceramide NG; steroid-based compounds such as oryzanol, betamethasone and dexamethasone dipropionate, and derivatives thereof; azole-based compounds such as clotrimazole, and derivatives thereof; benzopyran-based compounds such as griseofulvin and pranlukast, and derivatives thereof; macrolide-based compounds such as clarithromycin, and derivatives thereof; naphthalene-based compounds such as (S)-(+)-naproxen, and derivatives thereof; oxazole-based compounds such as oxaprozin, and derivatives thereof; indole-based compounds such as indomethacin, and derivatives thereof; oil-soluble aromatic carboxylic acid-based compounds such as propyl gallate, and derivatives thereof; phthalocyanine-based compounds such as copper phthalocyanine, and derivatives thereof; water-soluble aromatic carboxylic acid-based compounds such as gallic acid, and derivatives thereof; pyrazole-based compounds such as celecoxib, and derivatives thereof; carboxylic acid-based compounds such as ibuprofen, and derivatives thereof; aniline-based compounds such as acetaminophenone, and derivatives thereof; biphenyl-based compounds such as felbinac, and derivatives thereof; benzophenone-based compounds such as fenofibrate, and derivatives thereof; and phenol-based compounds such as propofol, and derivatives thereof; and preferably include benzoquinone-based compounds such as coenzyme Q10, and derivatives thereof; taxane-based compounds such as docetaxel and paclitaxel, and derivatives thereof; limonoids such as azadirachtin; alkylsulfonate-based compounds such as busulfan, and derivatives thereof; and water-soluble vitamins such as ascorbic acid.

<Dispersion Medium>

Examples of the dispersion medium of the present invention include, but are not particularly limited to, a solvent in which the poorly soluble substance is either insoluble or hardly soluble in the dispersion medium, and includes solvents corresponding to "slightly soluble", "very slightly soluble" and "hardly soluble" mentioned in The Japanese Pharmacopoeia 17th edition to the poorly soluble substance. Examples of the dispersion medium in the present invention include a solvent in which a solubility (poorly soluble substance/dispersion medium) of the poorly soluble substance is 10 mg/mL or less at 20±5°C. From the viewpoint of reducing side effects, examples of the dispersion medium include water, a low-toxicity dispersion medium, a solvent in which any appropriate toxicity data is not found, or a combination thereof in "Guideline for Pharmaceuticals (PMSB/ELD Notification No. 307, March 30, 1998)". The dispersion medium of the present invention is preferably water, ethanol, propylene glycol, 1-butanol, animal and vegetable fats and oils, tetrahydrofuran, squalane, or a combination thereof, although it depends on the type of the poorly soluble substance, more preferably water, ethanol, propylene glycol, 1-butanol, or a combination thereof, and still more preferably water, propylene glycol, 1-butanol, or a combination thereof.

The dispersion medium can be appropriately selected based on the type of the poorly soluble substance. For example, when the poorly soluble substance is a polyphenol-based compound such as curcumin, or a derivative thereof; a fat-soluble vitamin such as ascorbyl palmitate or tocopherol; sphingolipid such as ceramide NP or ceramide NG; a steroid-based compound such as oryzanol, betamethasone or dexamethasone dipropionate, or a derivative thereof; an oil-soluble aromatic carboxylic acid ester-based compound such as propyl gallate, or a derivative thereof; a phthalocyanine-based compound such as copper phthalocyanine, or a derivative thereof; a pyrazole-based compound such as celecoxib, or a derivative thereof; a benzoquinone-based compound such as coenzyme Q10, or a derivative thereof; a taxane-based compound such as docetaxel or paclitaxel, or a derivative thereof; limonoid such as azadirachtin; or an alkylsulfonate-based compound such as busulfan, or a derivatives thereof, examples of the dispersion medium include water, propylene glycol, glycerin, and the like, of which water is preferable. For example, the poorly soluble substance is a benzopyran-based compound such as griseofulvin, pranlukast, or a derivative thereof; a macrolide-based compound such as clarithromycin, or a derivative thereof; an azole-based compound such as clotrimazole, or a derivative thereof; a naphthalene-based compound such as (S)-(+)-naproxen, or a derivative thereof; an oxazole-based compound such as oxaprozin, or a derivative thereof; an indole-based compound such as indomethacin, or a derivative thereof; a carboxylic acid-based compound such as ibuprofen, or a derivative thereof; an aniline-based compound such as acetaminophenone, or a derivative thereof; a biphenyl-based compound such as felbinac, or a derivative thereof; a benzophenone-based compound such as phenofibrate, or a derivative thereof; or a phenol-based compound such as propofol, or a derivative thereof, the dispersion medium is preferably water. For example, when the poorly soluble substance is a water-soluble vitamin such as ascorbic acid, examples of the dispersion medium include propylene glycol, 1-butanol, animal and vegetable fats and oils, and the like, of which propylene glycol, 1-butanol and animal and vegetable fats and oils are preferable. For example, when the poorly soluble substance is a water-soluble aromatic carboxylic acid-based compound such as gallic acid, or a derivative thereof, examples of the dispersion medium include animal and vegetable fats and oils, squalene, and the like, of which animal and vegetable fats and oils are preferable.

<Good Solvent>

The good solvent of the present invention is not particularly limited, but it is necessary that the solubility of the poorly soluble substance is larger than that of the above dispersion medium. Examples of the good solvent in the present invention include a solvent in which a solubility (poorly soluble substance/dispersion medium) of the poorly soluble substance is more than 10 mg/mL or less at $20 \pm 5°C$, although it depends on the type of the poorly soluble substance, and the good solvent is preferably a solvent in which a solubility of a poorly soluble substance is 20 mg/mL or more, and more preferably a solvent in which a solubility of a poorly soluble substance is 30 mg/mL or more. The good solvent of the present invention may be a solvent in which a solubility of the poorly soluble substance is more than 10 mg/mL at $55 \pm 5°C$, preferably a solvent in which a solubility of the poorly soluble substance is 20 mg/mL or more, and more preferably a solvent in which a solubility of a poorly soluble substance is 30 mg/mL or more. Specific examples of the good solvent include water, alcohol-based solvents, ketone-based solvents, ether-based solvents, alcohol ether-based solvents, alcohol ester-based solvents, ester-based solvents, lactone-based solvents, non-polar solvents, aromatic solvents, organic solvents such as dimethylformamide, acetonitrile and dimethyl sulfoxide, and fats and oils. Examples of the alcohol-based solvent include methanol, ethanol, propanol, 2-propanol, propanediol, 2-methyl-1,3 propanediol, isopentyldiol, terpineol, isopentyldiol, ethylene glycol, propylene glycol, butylene glycol, dipropylene glycol, isoprene glycol, polyethylene glycol, polypropylene glycol, glycerin, and the like. Examples of the ketone-based solvent include acetone, methyl ethyl ketone, methyl isobutyl ketone, cyclohexanone, and the like. Examples of the ether-based solvent include diisopropyl ether, dibutyl ether, 1,4-dioxane, crown ether, tetrahydrofuran, and the like. Examples of the alcohol ether-based solvent include methoxyethanol, ethylene glycol monomethyl ether, ethylene glycol monoethyl ether, propylene glycol monomethyl ether, propylene glycol monoethyl ether, and the like. Examples of the alcohol ester-based solvent include ethylene glycol acetate, propylene glycol monomethyl ether acetate, and the like. Examples of the ester-based solvent include ethyl acetate, butyl acetate, and the like. Examples of the lactone-based solvent include butyl lactone, and the like.

Examples of the non-polar solvent include hexane, cyclohexane, squalane, liquid paraffin, and the like. Examples of the aromatic solvent include toluene, xylene, acetophenone, and the like. Examples of the fats and oils include natural fats and oils, and specific examples thereof include animal and vegetable oils, such as salad oil, camellia oil, palm oil, rapeseed oil, olive oil, safflower oil and linseed oil. These may be used in combination of two or more types. The good solvent is preferably a ketone-based solvent, an alcohol-based solvent, an ether-based solvent, water, or a combination thereof, and more preferably acetone, ethanol, 2-propanol, propylene glycol, tetrahydrofuran, water, or a combination thereof, although it depends on the type of the poorly soluble substance. When the good solvent is mixed with the dispersion medium, the boiling point of the good solvent is preferably lower than that of the dispersion medium so that it can be removed by distillation. Since dimethylformamide, acetonitrile, methanol, ethylene glycol, 1,4-dioxane, methoxyethanol, hexane, cyclohexane, toluene and xylene are solvents corresponding to solvents showing toxicity of a certain level or higher (class 2) according to Residual Solvent Guidelines for Pharmaceuticals (Non-Patent Document 1), it is necessary to pay attention to the residual amount at this stage.

The good solvent can be appropriately selected based on the type of the poorly soluble substance. For example, when the poorly soluble substance is a polyphenol-based compound such as curcumin, or a derivative thereof; a fat-soluble vitamin such as ascorbyl palmitate or tocopherol; sphingolipid such as ceramide NP or ceramide NG; a steroid-based compound such as oryzanol, betamethasone or dexamethasone dipropionate, or a derivative thereof; an oil-soluble aromatic carboxylic acid ester-based compound such as propyl gallate, or a derivative thereof; a phthalocyanine-based compound such as copper phthalocyanine, or a derivative thereof; a pyrazole-based compound such as celecoxib, or a derivative thereof; a benzoquinone-based compound such as coenzyme Q10, or a derivative thereof; a taxane-based compound such as docetaxel or paclitaxel, or a derivative thereof; limonoid such as azadirachtin; or an alkylsulfonate-based compound such as busulfan, or a derivative thereof, examples of the good solvent include alcohol-based solvents, ketone-based solvents, ether-based solvents, and the like, of which ethanol, 2-propanol, propylene glycol, acetone and tetrahydrofuran are preferable. For example, when the poorly soluble substance is a water-soluble vitamin such as ascorbic acid, examples of preferable good solvent include water, and the like. For example, when the poorly soluble substance is a water-soluble aromatic carboxylic acid-based compound such as galvanic acid, or a derivative thereof, examples of the good solvent include alcohol-based solvents, water, and the like, of which ethanol and water are preferable.

Further, according to another aspect of the present invention, when the poorly soluble substance is a polyphenol-based compound such as curcumin, or a derivative thereof; a fat-soluble vitamin such as ascorbyl palmitate or tocopherol; sphingolipid such as ceramide NP or ceramide NG; a taxane-based compound such as docetaxel or paclitaxel, or a derivative thereof; or limonoid such as azadirachtin, examples of the good solvents include alcohol-based solvents, ether-based solvents, and the like, of which ethanol and tetrahydrofuran are preferable. When the poorly soluble substance is limonoid such as azadirachtin, the good solvent is preferably an alcohol-based solvent, more preferably ethanol, 2-propanol or propylene glycol, and still more preferably ethanol. When the poorly soluble substance is a phthalocyanine-based compound such as copper phthalocyanine, or a derivative thereof; a pyrazole-based compound such as celecoxib, or a derivative thereof; a benzoquinone-based compound such as coenzyme Q10, or a derivative thereof; or an alkylsulfonate-based compound such as busulfan, or a derivative thereof, examples of the good solvent include ketone-based solvents, and the like, of which acetone is preferable.

Further, according to still another aspect of the present invention, when the poorly soluble substance is a steroid-based compound such as oryzanol, betamethasone or dexamethasone dipropionate, or derivatives thereof, examples of the good solvent include alcohol-based solvents, ketone-based solvents, and the like, of which ethanol and acetone are preferable. When the poorly soluble substance is oryzanol, more preferable good solvent is ethanol. When the poorly soluble substance is betamethasone or dexamethasone dipropionate, more preferable good solvent is acetone. When the poorly soluble substance is a benzopyran-based compound such as griseofulvin or pranlukast, or a derivative thereof, examples of the good solvent include ketone-based solvents and ether-based solvents, of which acetone and tetrahydrofuran are preferable. When the poorly soluble substance is griseofulvin, more preferred solvent is acetone. When the poorly soluble substance is pranlukast, more preferable good solvent is tetrahydrofuran. When the poorly soluble substance is a macrolide-based compound such as clarithromycin, or a derivative thereof, examples of the good solvent include ketone-based solvent, of which acetone is preferable. When the poorly soluble substance is an azole-based compound such as clotrimazole, or a derivative thereof; a naphthalene-based compound such as (S)-(+)-naproxen, or a derivative thereof; an oxazole-based compound such as oxaprozin, or a derivative thereof; an indole-based compound such as indomethacin, or a derivative thereof; an oil-soluble aromatic carboxylic acid-based compound such as propyl gallate, or a derivative thereof; a phthalocyanine-based compound such as copper phthalocyanine, or a derivative thereof; a pyrazole-based compound such as celecoxib, or a derivative thereof; a carboxylic acid-based compound such as ibuprofen, or a derivative thereof; an aniline-based compound such as acetaminophenone, or a derivative thereof; a biphenyl-based compound such as felbinac, or a derivative thereof; a benzophenone-based compound such as phenofibrate, or a derivative thereof; or a phenol-based compound such as propofol, or a derivative thereof, examples of the good solvent include alcohol-based solvents, and the like, of which ethanol is preferable.

The content of the poorly soluble substance of the present invention is not particularly limited, but can be set at, for example, 0.001 to 70% by mass, preferably 0.01 to 60% by mass, and still more preferably 0.1 to 50% by mass, based on the entire solution containing the good solvent, the poorly soluble substance and the surfactant.

The content of the poorly soluble substance in the dispersion of the present invention is not particularly limited, but can be set at, for example, 0.001 to 50% by mass, preferably 0.001 to 30% by mass, more preferably 0.01 to 20% by mass, and still more preferably 0.1 to 10% by mass, based on the total amount of the dispersion.

<Surfactant>

Examples of the surfactant include those to be used, or to be used in future in pesticides, pharmaceuticals, quasi-drugs, cosmetics or foods. The surfactant is not particularly limited, but any of anionic surfactants, cationic surfactants, amphoteric surfactants and nonionic surfactants can be used. These surfactants may be either a low-molecular weight type or a high-weight type.

Examples of the anionic surfactant include sulfate esters such as alkyl sulfate ester, alkyl ether sulfate ester, polyoxyethylene alkyl ether sulfate ester, polyoxyalkylene ether sulfate ester, polyoxyethylene aryl ether sulfate ester and amide ether sulfate ester, and salts thereof; phosphate esters such as alkyl phosphate ester, aryl phosphate ester, polyoxyethylene alkyl ether phosphate and polyoxyethylene aryl ether phosphate, and salts thereof; phosphoric acids such as alkylphosphoric acid, arylphosphoric acid, polyoxyethylene alkyl ether phosphoric acid and polyoxyethylene aryl ether phosphoric acid, and salts thereof; sulfonic acids such as alkylbenzene sulfonic acid, olefin sulfonic acid and dialkyl sulfosuccinic acid ester, and salts thereof; carboxylic acids such as alkylcarboxylic acid, arylcarboxylic acid, polyoxyethylene alkyl ether carboxylic acid and polyoxyethylene aryl ether carboxylic acid, and salts thereof; sulfocarboxylic acids such as polyoxyethylene alkyl sulfosuccinate, and salts thereof; ananinates and salts thereof; fatty acid methylalanine, and salts thereof; sarcosine derivatives; taurine derivatives, and the like. Examples of the dialkyl sulfosuccinic acid ester include sodium dialkyl sulfosuccinate, and specific examples thereof include NEOCOL YSK (manufactured by Dai-ichi Kogyo Seiyaku Co., Ltd.).

Examples of the cationic surfactant include alkyltrimethylammonium salts, alkyl polyethylene glycol trimethylammonium salts, alkyldimethylbenzylammonium salts, alkylamidoamines; amines such as alkylamine and aromatic amine, and salts thereof; quaternary ammonium salts, and the like.

Examples of the amphoteric surfactant include alkyl benzine type, animal and vegetable oil fatty acid alkyl benzine type, alkyl amide betaine type, animal and vegetable oil fatty acid alkyl amide benzine type, alkylamine oxide type, amino acid-based amphoteric surfactant type, amine oxide type, fatty acid amide alkyldimethylamine oxide type, hydroxyalkyl hydroxyalkyl sarcosine type and imidazolinium betaine type surfactants.

Examples of the nonionic surfactant include glycol hydroxypropyl ether type, alkylol amide type, polyoxyethylene alkyl ether type, polyoxyethylene aryl ether type, polyoxyalkylene derivative, polyoxyethylene alkyl ester type, polyoxyethylene aryl ester, polyoxyethylene hydrogenated castor oil type, polyoxyethylene hydrogenated castor oil ether type, polyoxyethylene fatty acid ethanolamide type, polyoxyethylene animal and vegetable oil fatty acid ethanolamide type, polyoxypropylene fatty acid isopropanolamide type, polyoxypropylene animal and vegetable oil fatty acid isopropanolamide type, fatty acid ethanolamide type, animal and vegetable oil fatty acid ethanolamide type, modified animal and vegetable oil fatty acid ethanolamide type, fatty acid isopropanolamide type, animal and plant fatty acid isopropanolamide type, polyoxyethylene derivative, polyoxyethylene polyoxypropylene glycol type, polyoxyethylene fatty acid ester type, polyoxyethylene fatty acid ether type, polyoxyethylene glycol fatty acid ester type, glycerin fatty acid ester type, polyoxyethylene glycerin ester type, polyoxyethylene polyoxypropylene alkyl ether type, polyoxyethylene sorbit fatty acid ester type, sorbitan fatty acid ester type, sucrose fatty acid ester type, polyoxyethylene sorbitan fatty acid ester type, polyoxyethylene sorbitol fatty acid ester type, deodorized polyether type, polyoxyethylene alkylamine type, polyoxyethylene animal and vegetable oil alkylamine type, polyoxyethylene caprylylamine type, polyoxyethylene animal and vegetable oil caprylylamine type, polyoxyethylene arylamine type, alkylalkanolamide type and alkyl polyglucoside type surfactants. Examples of the polyoxyethylene fatty acid ester type surfactant include polyoxyethylene distearate, and specific examples thereof include EMANON 3299RV (manufactured by Kao Corporation), and the like. Specific examples of the polyoxyethylene fatty acid ether type surfactant include EMULGEN 123P (manufactured by Kao Corporation), and the like. Specific examples of the polyoxyethylene polyoxypropylene alkyl ether type surfactant include PBC-34 (manufactured by NIPPON SURFACTANT INDUSTRIES CO., LTD.), and the like. Specific examples of the sucrose fatty acid ester type surfactant include COSMELIKE L-160A (manufactured by Dai-ichi Kogyo Seiyaku Co., Ltd.), and the like.

Examples of the high-molecular weight type surfactant include polycarboxylic acids and salts thereof; polyacrylic acids and salts thereof; acrylic acid-maleic acid copolymers, polymethacrylic acid, and salts thereof; polyamides, polyesters, polylactic acids, polyallylamines and salts thereof; naphthalenesulfonic acid formarin condensates and salts thereof; vinyl acetate, polyvinylpyridone, vinyl acetate-vinylporolidone copolymers, polyvinyl alcohol, polyvinyl alcohol-polyethylene glycol copolymers; polyalkylene polyamine alkylene oxide adducts; polyalkylene polyimine alkylene oxide adduct, polyvinylcaprolactam-polyvinyl acetate-polyethylene glycol graft copolymers, polyoxyethylene-pooxypropylene glycol, polyethyleneimine, styrene-maleic acid copolymers and salts thereof, polydiallyldimethylammonium and salts thereof; polyethylene glycol, polypropylene glycol, polyoxyethylene polyoxypropylene glycol condensates; sugar derivatives such as sucrose alkyl ester, sucrose aryl ester, alginic acid, agar, gum arabic, xanthan gum, tragant gum, starch, alkyl cellulose, hydroxyalkyl cellulose, carboxycellulose, carboxyalkylcellulose and cationized cellulose; and proteins such as gelatin and collagen.

The above surfactants may be used alone, or may be optionally used in two or more types thereof.

Examples of preferable surfactant include anionic surfactants and nonionic surfactants, of which dialkyl sulfosuccinic acid ester salts, polyoxyethylene fatty acid ester type surfactants, polyoxyethylene fatty acid ether type surfactants, polyoxyethylene polyoxypropylene alkyl ether type surfactants and sucrose fatty acid ester type surfactants are more preferable.

The content of the surfactant is not particularly limited, but can be set, for example, 0.1 to 80% by mass, preferably 1 to 70% by mass, and more preferably 2 to 60% by mass, based on the entire solution containing the good solvent, the poorly soluble substance and the surfactant.

In the present invention, the use of the surfactant is advantageous in that ultrafine particles can be stably dispersed in a non-toxic or low-toxic dispersion medium such as water or ethanol. While not intending to be bound by any specific theory, it is considered that the surfactant contributes to stable dispersion of the precipitated ultrafine particles in the rapidly cooled good solvent during the manufacturing process.

The mass ratio of the poorly soluble substance to the surfactant (poorly soluble substance:surfactant) of the present invention is, for example, in a range of about 1:0.1 to 500, preferably about 1:0.1 to 300, and more preferably about 1:1 to 300.

Manufacturing Method>

The method for manufacturing a poorly soluble substance dispersion of the present invention includes preparing a solution containing a good solvent and the poorly soluble substance and the surfactant dissolved in the good solvent; rapidly cooling the solution to a temperature at which the poorly soluble substance precipitates in the solution at a temperature lowering rate of 100 to 4,000°C/second to produce ultrafine particles with a particle size of a nano-order level formed of the poorly soluble substance in the good solvent; and then separating the good solvent from a mixed solution of the solution and the dispersion medium after mixing the solution and the dispersion medium, or mixing the dispersion medium to the solution after separating the good solvent from the solution. The means for rapidly cooling the solution is not particularly limited. As an example of the means for rapid cooling, an embodiment using a micro heat exchanger will be described below with reference to the drawings.

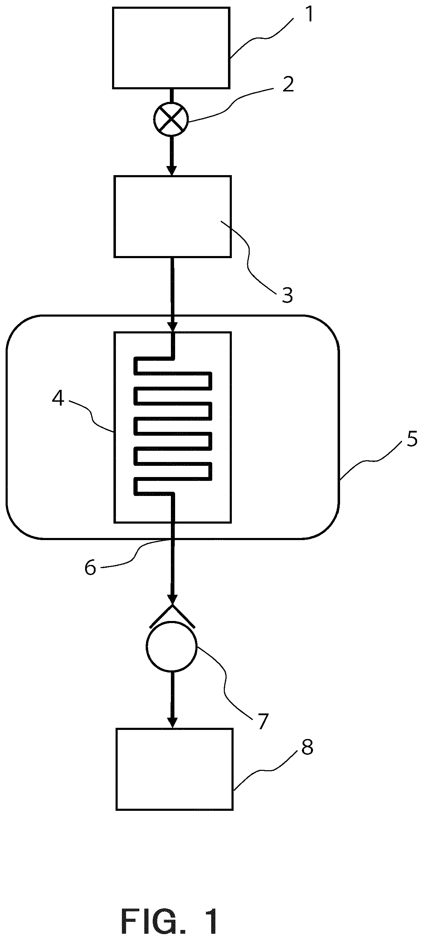

FIG. 1 shows a flow chart of the manufacturing method according to the present invention. First, as a raw material liquid, a solution 1 containing a good solvent and a poorly soluble substance and a surfactant dissolved in the good solvent is prepared (hereinafter also referred to as raw material liquid 1). The raw material liquid 1 can be prepared by dissolving a poorly soluble substance and a surfactant in a good solvent. It is preferable that a container for containing the raw material liquid is equipped with a refluxer.

The raw material liquid 1 is supplied to a warmer 3 from the container containing the raw material liquid 1 mentioned above. The flow rate of the supplied raw material liquid, i.e., the flow rate of the raw material liquid flowing through a flow path is 0.1 µL/minute to 5 L/minute, preferably 0.5 mL/minute to 2 L/minute, and more preferably 10 mL/minute to 500 mL/minute.

A liquid feed pump 2 can be used to feed the raw material liquid 1 to the warmer 3. As the liquid feed pump, a syringe type pump can be used, but a plunger pump capable of suppressing the pulsation of the liquid feed and continuously transferring an accurate capacity can be preferably used. The temperature of the warmer 3 is adjusted to, for example, 20 to 300°C, and preferably 30 to 180°C Therefore, in the warmer 3, the temperature of the raw material liquid 1 is adjusted to 20 to 300°C, and preferably 30 to 180°C It is possible to use, as the warmer 3, a device for preliminarily heating so that the poorly soluble substance does not precipitate before moving to the micro exchanger can be used.

The equivalent diameter of the flow path connecting each of the raw material liquid 1, the liquid feed pump 2, the warmer 3, the micro heat exchanger 4, the check valve 7 and the container 8 is preferably 0.1 to 5 mm.

The raw material liquid heated by passing through the warmer 3 is supplied directly to the micro heat exchanger 4 without being exposed to the outside air. The micro heat exchanger 4 is a heat exchanger having a micro flow path for the purpose of performing heat exchange in a micro space. The equivalent diameter of the flow path of the micro heat exchanger used in the present invention is 5 mm or less, preferably 0.5 to 3 mm, and more preferably 0.5 to 2 mm. The shape of the cross section of the flow path is not particularly limited as long as the flow path diameter is within this range, and examples of the flow path include capillary-shaped, tube-shaped and substrate-shaped flow paths. The length of the flow path can be appropriately changed depending on the object to be cooled, the cooling conditions, the flow rate, and the like, and is about 5 cm to 30 m, and more preferably 5 cm to 100 cm.

It is possible to use, as the micro heat exchanger, for example, a continuous micro heat exchanger in which a plurality of micro heat exchangers are connected in series, or a micro heat exchanger arranged in parallel. By increasing the number of micro heat exchangers, the amount of ultrafine particles obtained per unit time can be increased. The shape of the micro heat exchanger is not particularly limited, but those with known shape can be used, and micro heat exchangers with any shape, such as a T-shaped or Y-shaped micro heat exchanger may be used.

Examples of the material of the micro heat exchanger include, but are not particularly limited to, conventionally used inorganic materials such as glass, quartz, ceramic and silicon; and organic materials, for example, polyolefin resins, polyacrylic resins, polycarbonate resins, polyester resins, silicone resins such as polydimethylsiloxane, thermoplastic resins such as a polyether ether ketone resin, a thermoplastic polyimide resin and a fluororesin, and thermosetting resins such as an epoxy resin, a phenol resin and a thermosetting polyimide resin. Of these, glass and quartz are preferable from the viewpoint of the chemical resistance, the heat resistance, and the like. When the inorganic material is used as the material of the micro heat exchanger, a micron-order flow path can be formed by optical lithography technology well known in the field of semiconductor processing technology, or pulse laser processing. When the organic material is used, a micron-order flow path can be formed by an optical patterning technology of irradiating with ultraviolet rays to cure the material.

In the micro heat exchanger 4, ultrafine particles of a poorly soluble substance can be precipitated by rapid cooling, preferably high-speed rapid cooling, and more preferably ultra-high-speed rapid cooling of the heated raw material liquid.

Precipitation by rapid cooling utilizes the difference in solubility of the poorly soluble substance in a good solvent depending on the temperature, and is preferably also referred to as microcrystallization. The temperature difference $\Delta t$ in the rapid cooling operation can be determined as (outlet temperature in the warmer 3) - (outlet temperature in the micro heat exchanger 4). The temperature difference $\Delta t$ is 5 to 400°C, preferably 5 to 250°C, and more preferably 20 to 200°C

The temperature lowering rate can be calculated as $\Delta t$/(cooling time), and it is necessary to set it at 100 to 4,000°C/sec from the viewpoint of microparticulation by rapid cooling. The temperature lowering rate is preferably 200 to 3,000°C/sec, and more preferably 300 to 2,000°C/sec.

The temperature at which the poorly soluble substance precipitates in the solution can be set based on the type of the poorly soluble substance, the concentration of the poorly soluble substance in the solution and the type of the good solvent, and is preferably lower than the temperature at which the poorly soluble substance begins to precipitate in the solution, and more preferably 10°C or lower than the temperature at which the poorly soluble substance begins to precipitate. For example, when coenzyme Q10 is used as the poorly soluble substance and acetone is used as the good solvent, the temperature is preferably 20°C or higher lower than the temperature at which the poorly soluble substance begins to precipitate. Here, the beginning of precipitation of particles can be visually observed since the particles precipitate to produce turbidity when the temperature of the solution of the poorly soluble substance placed in a constant temperature oil tank is gradually lowered.

The temperature of the flow path in the micro heat exchanger must be set at the temperature equal to or lower than the temperature at which the poorly soluble substance begins to precipitate in the solution, and is preferably 10°C or lower than the temperature at which the poorly soluble substance begins to precipitate. In order to set at such temperature condition, the micro heat exchanger 4 may be cooled by a coolant, and the micro heat exchanger 4 may be arranged in the container 5 containing the coolant. Examples of the coolant include liquid nitrogen, liquid helium, dry ice, ice, and the like, or a solvent such as ethylene glycol cooled with liquid nitrogen, liquid helium, dry ice, ice, and the like. The flow rate of the solution flowing through the flow path is, for example, 0.1 μL/minute to 5 L/minute, preferably 0.5 mL/minute to 2 L/minute, and more preferably 10 mL/minute to 500 mL/minute. The raw material liquid passes through the micro heat exchanger having the flow path length as mentioned above at such flow rate, thus enabling precipitation of ultrafine particles. When the micro heat exchanger is used, the turbulence can be increased (the turbulence can be created). Further, since the micro heat exchanger has a small flow path diameter as mentioned above and the surface area of the flow path to the flow rate (i.e., volume) is extremely large, it is possible to strictly control the temperature of the raw material liquid passing through the flow path held at a predetermined temperature in the micro heat exchanger.

The pressure of the flow path in the micro heat exchanger is preferably 3.0 MPa or less, and more preferably 0.5 to 2.0 MPa, from the viewpoint of improving the solubility of the poorly soluble substance in the good solvent or preventing boiling. In order to set at such pressure, the pressure may be adjusted by the check valve 7.

The liquid in which the ultrafine particles are precipitated in the micro heat exchanger is taken out from the outlet and held in the container 8.

Examples of the method for manufacturing a dispersion in which the poorly soluble substance is dispersed in the dispersion medium with a particle size of a nano-order level from the liquid held in the container 8 include the following methods (i) and (ii).

(i) After mixing the dispersion medium with the liquid held in the container 8, the good solvent is distilled off from the mixed solution of the solution and the dispersion medium, and preferably separated by concentration under reduced pressure to obtain a dispersion in which the poorly soluble substance is dispersed in the dispersion medium with a particle size of a nano-order level. Here, the mass ratio of the liquid held in the container 8 to the dispersion medium (liquid held in the container 8:dispersion medium) is preferably 1:0.1 to 10.

An excipient is optionally added to the dispersion obtained above, and then the dispersion medium is removed to obtain a powder containing ultrafine particles of a poorly soluble substance. Examples of the method for removing the dispersion medium include drying, and freeze-drying, spray-drying, vacuum-drying and hot-air drying are preferable, and freeze-drying or vacuum-drying is more preferable. The mass ratio of the excipient to be mixed to the dispersion (dispersion:excipient) is not particularly limited, but is, for example, in a range of about 1:1 to 100, and preferably 1:5 to 20. The mass ratio of the excipient to be mixed to the ultrafine particles of the poorly soluble substance (ultrafine particles of the poorly soluble substance:excipient) is not particularly limited, but is, for example, in a range of about 1:1 to 500, preferably about 1:10 to 400, and more preferably about 1:50 to 300. Here, it is preferable that the powder containing ultrafine particles of a poorly soluble substance is allowed to contain a binder from the viewpoint of preventing scattering. Therefore, in the preparation of the powder containing ultrafine particles of a poorly soluble substance, it is preferable to further mix a binder in the dispersion.

(ii) The good solvent is separated from the liquid held in the container 8 and then a dispersion medium is mixed to obtain a dispersion in which a poorly soluble substance is dispersed in the dispersion medium with a particle size of a nano-order level. Here, examples of the method for separating the good solvent include freeze-drying, spray-drying, vacuum-drying and hot-air drying, and freeze-drying or vacuum-drying is preferable. The mass ratio of the liquid held in the container 8 to the dispersion medium (liquid held in the container 8:dispersion medium) is preferably 1:0.1 to 10.

Similar to (i), an excipient is optionally added to the dispersion obtained above, and then the dispersion medium is removed by drying to obtain a powder containing ultrafine particles of a poorly soluble substance. Further, in (ii), a powder containing ultrafine particles of a poorly soluble substance can be obtained by separating a good solvent from the liquid held in the container 8. Here, as in (i), it is preferable that a binder is allowed to contain in the dispersion or the good solvent from the viewpoint of preventing scattering.

<Ultrafine Particles>

Ultrafine particles of the poorly soluble substance obtained by the above method of the present invention are preferably microcrystals (crystal state). The ultrafine particles are considered to have a structure in which a molecule of a surfactant is adhered to the surface of microcrystalline particles of the poorly soluble substance.

The average particle size of the ultrafine particles of the poorly soluble substance in the dispersion of the present invention is not particularly limited, but is, for example, 0.5 to 300 nm, preferably 0.8 to 200 nm, more preferably 1 to 100 nm, still more preferably 1 to 10 nm, and further preferably 1 to 5 nm. The average particle size (based on the number of particles) refer to the average of the particle sizes obtained by arbitrarily measuring the area of 200 ultrafine particles from TEM images observed using a known transmission electron microscope (TEM) as the diameter of a perfect circle with the same area as that of the particles.

Here, the average particle size of the ultrafine particles of the poorly soluble substance in the dispersion can be appropriately set based on the conditions such as the type of components in the dispersion and mixing ratios thereof so that those skilled in the art can understand.

The coefficient of variation of the particle size of the ultrafine particles of the poorly soluble substance is, for example, 5 or less, preferably 1 or less, more preferably 0.5 or more, still more preferably 0.2 or less, yet more preferably 0.1 or less, further preferably 0.05 or less, and still further preferably 0.01 or more and 0.05 or less. Therefore, as mentioned above, it is also a feature of the present invention that the particle size is very uniform.

The coefficient of variation CV of the particle size of the ultrafine particles of the poorly soluble substance is a value obtained by dividing the standard deviation s by the average particle size, and is represented by the following equation.

[Equation 1]

$$ CV = \frac{s}{x_{ave}} = \frac{\sqrt{s^2}}{x_{ave}} = \frac{1}{x_{ave}} \sqrt{\frac{1}{n} \sum_{i=1}^{n} (x_i - x_{ave})^2} $$

In the above equation, s represents the standard deviation, $s^2$ represents the dispersion, n represents the total number of particles, $x_i$ represents the particle size of each particle, and $x_{ave}$ represents the average particle size.

The fine particles having a particle size of a nano-order level obtained as mentioned above can stably maintain a dispersed state even in a dispersion medium (poor solvent) such as water by Brownian motion. Further, the dispersion in which the ultrafine particles are dispersed in the dispersion medium is clear and not cloudy since the fine particles have a particle size of a nano-order level.

The fine particles of the poorly soluble substance in the powder containing ultrafine particles of a poorly soluble substance obtained by removing the dispersion medium also have the same average particle size and shape as those of the ultrafine particles of the poorly soluble substance in the dispersion. Further, by adding an excipient such as mannitol to a dispersion of ultrafine particles of a poorly soluble substance, followed by drying, it is possible to obtain a powder containing ultrafine particles of a poorly soluble substance adsorbed on the excipient.

The mass ratio of the poorly soluble substance to the surfactant (poorly soluble substance:surfactant) in the ultrafine particles of the present invention is, for example, in a range of about 1:0.1 to 500, preferably about 1:0.1 to 300, and still more preferably about 1:1 to 300.

<Powder containing Ultrafine Particles of Poorly Soluble Substance>

The powder containing ultrafine particles of a poorly soluble substance may contain an excipient together with the ultrafine particles of a poorly soluble substance.

The content of the ultrafine particles of the poorly soluble substance in the powder containing ultrafine particles of a poorly soluble substance of the present invention is not particularly limited, but can be set at, for example, 0.001 to 30% by mass or 95 to 100% by mass, preferably 0.01 to 20% by mass or 96 to 100% by mass, and more preferably 0.1 to 10% by mass or 98 to 100% by mass, based on the total amount of the powder containing ultrafine particles of a poorly soluble substance.

The content of the excipient in the powder containing ultrafine particles of a poorly soluble substance of the present invention is not particularly limited, but can be set at, for example, 70 to 99.9% by mass or 0% by mass, preferably 70 to 99.8% by mass or 0% by mass, and more preferably 80 to 99.7% by mass or 0% by mass, based on the total amount of the powder containing ultrafine particles of a poorly soluble substance.

The mass ratio of the ultrafine particles of the poorly soluble substance to the excipient (ultrafine particles of the poorly soluble substance:excipient) in the powder containing ultrafine particles of a poorly soluble substance of the present invention is, for example, in a range of about 1:1 to 500, preferably about 1:10 to 400, and more preferably about 1:50 to 300, when the powder containing ultrafine particles of a poorly soluble substance of the present invention contains an excipient.

Examples of the excipient used in the present invention include, but are not particularly limited to, orally acceptable or pharmaceutically acceptable excipients. Examples of such excipients include mannitol, fructose, sorbitol, lactose, dextrin, cyclodextrin, cellulose, starch, sucrose; and inorganic substances such as calcium carbonate, calcium phosphate, talc and silica, of which mannitol and lactose are preferable.

<Composition>

The dispersion and the powder containing ultrafine particles of a poorly soluble substance of the present invention can be directly used as a composition by optionally mixing with an orally acceptable or pharmaceutically acceptable additive. Examples of such additives include solvents, resolvents, solubilizers, lubricants, emulsifiers, isotonic agents, stabilizers, preservatives, antiseptics, surfactants, regulators, chelators, pH adjusters, buffers, excipients, thickeners, colorants, fragrances or flavors, and the like.

The composition of the present invention is not particularly limited, but pharmaceuticals including veterinary drugs, quasi-drugs, pesticides, cosmetics, foods, food additives or supplements are preferable.

The content of the poorly soluble substance mixed in the composition of the present invention is not particularly limited, but is, for example, 0.001 to 30% by mass, and preferably 0.01 to 20% by mass, based on the total amount of the composition.

The composition of the present invention can be prepared by a known method for mixing, dissolving, dispersing and suspending the above dispersion or power containing ultrafine particles of a poorly soluble substance, and optionally, an orally acceptable or pharmaceutically acceptable additive. Further, in the preparation of the composition of the present invention, the mixture, solution, dispersion, suspension, and the like prepared by the above method may be subjected to a homogenization treatment or a sterilization treatment as long as it does not interfere with the effect of the present invention.

The form of the composition of the present invention is not particularly limited as long as it does not interfere with the effect of the present invention, and may be solid, semi-solid or liquid, but is preferably solid or liquid.

The dosage form of the composition of the present invention is not particularly limited as long as it does not interfere with the effect of the present invention, and examples thereof include injections, tablets (e.g., uncoated tablets, sugar-coated tablets, film-coated tablets, enteric coated tablets, sustained-release tablets, orally disintegrating tablets, sublingual tablets, chewable tablets, etc.), capsules (e.g., hard capsules, soft capsules), elixirs, pills, dusts, powders, granules, solutions, troches, syrups, dry syrups, emulsions, suspensions, liquids, inhalants, aerosols, powder inhalants, suppositories, ointments, creams, gels, patches, cataplasms, lotions, drops, eye ointments, eye drops, nasal drops, and the like. Examples of the dosage form of the composition of the present invention are preferably injections, drops, tablets, capsules, pills, dusts, powders, granules, syrups, dry syrups, emulsions, solutions, suspensions, liquids, troches, and the like.

According to one embodiment, the subject to which the composition of the present invention is applied is not particularly limited as long as it does not interfere with the effect of the present invention, but is preferably a mammal, and more preferably a human. According to another aspect of the present invention, when the composition of the present invention is a pesticide, the subject to which the composition of the present invention is applied is not particularly limited as long as it does not interfere with the effect of the present invention, but examples thereof include plants, insects, arachnids, nematodes, bacteria, fungi, viruses, mammals such as mice, and the like.

EXAM PLES

The present invention will be described in more detail below by way of Examples, but the present invention is not limited to these Test Examples. Unless otherwise specified, the measuring method and unit of the present invention are in accordance with the provisions of the Japanese Industrial Standards (JIS).

Example 1: Preparation of Dispersion of Ultrafine Particles of Coenzyme 010

In a container equipped with a refluxer, 94.5 g of acetone was charged and 5 g of PBC-34 (manufactured by NIPPON SURFACTANT INDUSTRIES CO., LTD.) was added as a surfactant, followed by stirring. Thereafter, 0.5 g of coenzyme Q10 (Ubiquinone-10, manufactured by Wako Pure Chemical Industries, Ltd.) was added, followed by stirring to prepare a raw material liquid. The solubility of coenzyme Q10 in acetone at 25°C was 30 mg/mL or more.

The raw material liquid prepared as mentioned above was warmed to 50°C and then allowed to flow to a warmer with a liquid feed pump. The temperature at the outlet of the warmer of the heated raw material liquid was 130°C.

Next, the warmed raw material liquid was rapidly cooled using a micro heat exchanger. The temperature lowering rate determined from the flow rate, the equivalent diameter of the flow path, the length of the flow path, the temperature difference before and after cooling, and the like was 1,400°C/sec. The flow rate in the micro heat exchanger was 20 mL/minute. The temperature of the liquid determined by rapid cooling at the outlet of the micro heat exchanger was -10°C. The pressure in the piping of the micro heat exchanger was adjusted so as not to exceed 2.0 MPa. The liquid leaving the micro heat exchanger was a pale yellow transparent liquid.

To 100 mL of the liquid thus obtained, 100 mL of water was added and acetone was distilled off by concentration under reduced pressure to obtain a water dispersion of ultrafine particles of coenzyme Q10. The water dispersion thus obtained was pale yellow transparent. The solubility of coenzyme Q10 in water at 25°C was 0.01 mg/mL or less. Specifically, as the method for measuring the solubility, 100 mL of water was added to 1 mg of coenzyme Q10, followed by stirring at 25°C for 1 hour and further being left to stand for 1 hour. As a result, it was judged to have the above solubility because crystals were present.

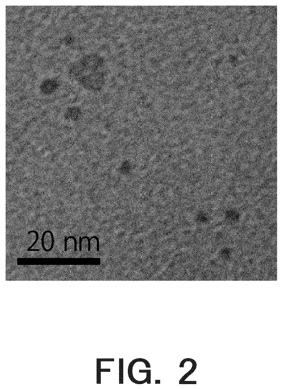

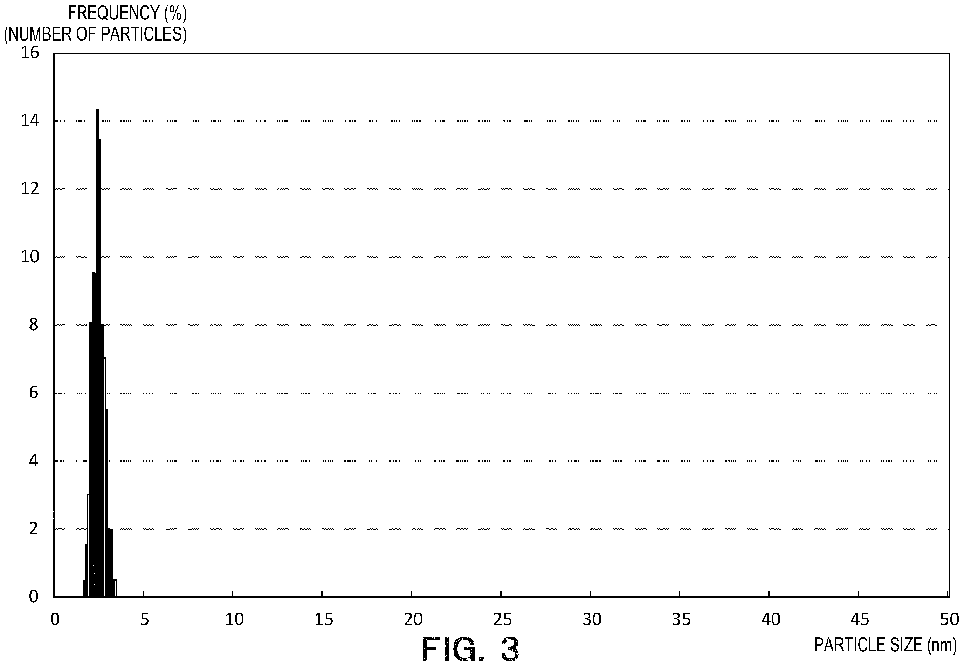

Using a transmission electron microscope (H-7650, Hitachi High-Technologies Corporation), the average particle size of the ultrafine particles of coenzyme Q10 in the water dispersion was measured as follows. First, the water dispersion of the ultrafine particles was placed on an observation grid and then dried to obtain a TEM observation sample. Using Mac-View (Mountech Co., Ltd.), the average particle size was calculated from the TEM image of the sample with the particle size of 200 ultrafine particles. At this time, the particle size of the ultrafine particles is determined as the diameter of a perfect circle with the same area as that of the particles. The average particle size distribution (based on the number of particles) was determined based on the particle size thus obtained, and then the average particle size and the standard deviation were calculated. As a result, the average particle size was 2.47 nm and the standard deviation was 0.1088 nm. FIG. 2 shows a part of TEM images. The particle size distribution thus obtained is shown in FIG. 3. Here, the particle size was uniform, and the shape of the particles was also uniform.

Example 1-1: Preparation of Powder containing Ultrafine Particles of Coenzyme 010

Water was removed from the water dispersion obtained in Example 1 to obtain a powder containing ultrafine particles of coenzyme Q10. Specifically, the pressure was reduced to 10 Torr using a rotary evaporator R-200 (manufactured by BUCHI) and the water dispersion was vacuum-dried at 40°C. As a result, a powder containing ultrafine particles of coenzyme Q10 was obtained.

Example 2: Preparation of Dispersion of Ultrafine Particles of Busulfan

In a container equipped with a refluxer, 94.5 g of acetone was charged and 5 g of COSMELIKE L-160A (manufactured by Dai-ichi Kogyo Seiyaku Co., Ltd.) was added as a surfactant, followed by stirring. Thereafter, 0.5 g of busulfan (manufactured by Wako Pure Chemical Industries, Ltd.) was added, followed by stirring to prepare a raw material liquid. The solubility of busulfan in acetone at 50°C was 30 mg/mL or more.

The raw material liquid prepared as mentioned above was warmed to 50°C and then allowed to flow to a warmer with a liquid feed pump. The temperature at the outlet of the warmer of the heated raw material liquid was 130°C.

Next, the warmed raw material liquid was rapidly cooled using a micro heat exchanger. The temperature lowering rate determined from the flow rate, the equivalent diameter of the flow path, the length of the flow path, the temperature difference before and after cooling, and the like was 1,400°C/sec. The flow rate in the micro heat exchanger was 20 mL/minute. The temperature of the liquid determined by rapid cooling at the outlet of the micro heat exchanger was -10°C. The pressure in the piping of the micro heat exchanger was adjusted so as not to exceed 2.0 MPa. The liquid leaving the micro heat exchanger was a colorless transparent liquid.

To 100 mL of the liquid thus obtained, 100 mL of water was added and acetone was distilled off by concentration under reduced pressure to obtain a water dispersion of ultrafine particles of busulfan. The water dispersion thus obtained was colorless transparent. The solubility of busulfan in water at 25°C was 0.01 mg/mL or less. The solubility was measured in the same manner as in Example 1, except that busulfan was used in place of coenzyme Q10.

The average particle size of the ultrafine particles of busulfan was measured in the same manner as in Example 1 using a transmission electron microscope. As a result, the average particle size was 3 nm. The standard deviation was 0.1063 nm.

Example 2-1: Preparation of Powder containing Ultrafine Particles of Busulfan

After adding an excipient to the water dispersion obtained in Example 2, water was removed to obtain a powder containing ultrafine particles of busulfan. Specifically, 10 g of mannitol was added to 1 g of the water dispersion, and then the pressure was reduced to 10 Torr using a rotary evaporator R-200 and the water dispersion was vacuum-dried at 40°C. As a result, a powder containing ultrafine particles of busulfan was obtained.

Reference Examples 2-1 and 2-2: Preparation of Dispersion of Particles of Busulfan without being subjected to Rapid Cooling Treatment

As Reference Example 2-1 a dispersion of particles of busulfan, which does not contain a surfactant and is not subjected to a rapid cooling treatment, was prepared. The dispersion of Reference Example 2-1 was prepared in the same manner as in Example 2, except that the surfactant was not used and warming with a warmer and rapid cooling were not performed.

As Reference Example 2-2 a dispersion of particles of busulfan, which is not subjected to a rapid cooling treatment, was prepared. The dispersion of Reference Example 2-2 was prepared in the same manner as in Example 2, except that warming with a warmer and rapid cooling were not performed.

Test Example 1: Stability Test of Dispersion of Ultrafine Particles of Busulfan

The dispersions of Example 2, Reference Example 2-1 and Reference Example 2-2 were charged in a glass container and then left to stand for 2 hours, respectively. After 2 hours, the solubility was visually confirmed and the laser irradiation was performed.

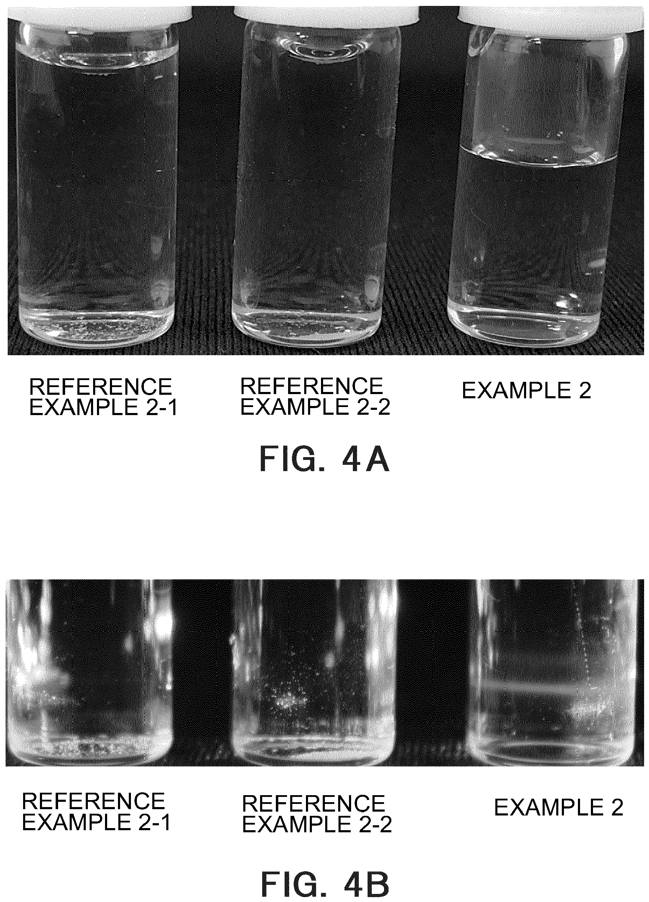

Here, a healthy panelist with visual acuity (visual acuity of 1.0 or more) visually confirmed the solubility by setting the distance between the dispersion and the eyes at 20 cm in a vertical direction under light having an illuminance of 300 to 2,000 lux. The solubility was confirmed by three panelists. As a result, the dispersion of Example 2 was colorless and transparent after being left to stand for 2 hours, and no precipitate was observed. On the other hand, in the dispersions of Reference Examples 2-1 and 2-2, a precipitate was observed after being left to stand for 2 hours. The results are shown in FIG. 4A.

Further, after being left to stand for 2 hours, the dispersion was irradiated using a laser pointer (wavelength of 520 nm). As a result, Tyndall phenomenon was confirmed in the dispersion of Example 2, but Tyndall phenomenon was not confirmed in the dispersions of Reference Examples 2-1 and 2-2. The results are shown in FIG. 4B.

Example 3: Preparation of Dispersion of Ultrafine Particles of Docetaxel

In a container equipped with a refluxer, 94.5 g of ethanol was charged and 5 g of EMULGEN 123P (manufactured by Kao Corporation) was added as a surfactant, followed by stirring. Thereafter, 0.5 g of docetaxel (manufactured by Tokyo Chemical Industry Co., Ltd.) was added, followed by stirring to prepare a raw material liquid. The solubility of docetaxel in ethanol at 60°C was 20 mg/mL or more.

The raw material liquid thus prepared as mentioned above was warmed to 50°C and then allowed to flow to a warmer with a liquid feed pump. The temperature at the outlet of the warmer of the warmed raw material liquid was 130°C

Next, the warmed raw material liquid was rapidly cooled using a micro heat exchanger. The temperature lowering rate determined from the flow rate, the equivalent diameter of the flow path, the length of the flow path, the temperature difference before and after cooling, and the like was 1,400°C/sec. The flow rate in the micro heat exchanger was 20 mL/minute. The temperature of the liquid determined by rapid cooling at the outlet of the micro heat exchanger was -10°C. The pressure in the piping of the micro heat exchanger was adjusted so as not to exceed 2.0 MPa. The liquid leaving the micro heat exchanger was a colorless transparent liquid.

To 100 mL of the liquid thus obtained, 100 mL of water was added and ethanol was distilled off by concentration under reduced pressure to obtain a water dispersion of ultrafine particles of docetaxel. The water dispersion thus obtained was colorless transparent. The solubility of docetaxel in water at 25°C was 0.01 mg/mL or less. The solubility was measured in the same manner as in Example 1, except that docetaxel was used in place of coenzyme Q10.

The average particle size of the ultrafine particles of docetaxel was measured in the same manner as in Example 1 using a transmission electron microscope. As a result, the average particle size was 3 nm. The standard deviation was 0.5811 nm.

Example 3-1 Preparation of Powder containing Ultrafine Particles of Docetaxel

Water was removed from the water dispersion obtained in Example 3 to obtain a powder containing ultrafine particles of docetaxel. Specifically, 10 g of mannitol was added to 1 g of the water dispersion, and then the pressure was reduced to 10 Torr using a rotary evaporator R-200 and the water dispersion was vacuum-dried at 40°C. As a result, a powder containing ultrafine particles of docetaxel was obtained.

Reference Examples 3-1 and 3-2: Preparation of Dispersion of Particles of Docetaxel without being subjected to Rapid Cooling Treatment

As Reference Example 3-1, a dispersion of particles of docetaxel, which does not contain a surfactant and is not subjected to a rapid cooling treatment, was prepared. The dispersion of Reference Example 3-1 was prepared in the same manner as in Example 3, except that the surfactant was not used and warming with a warmer and rapid cooling were not performed.

As Reference Example 3-2, a dispersion of particles of docetaxel particles, which is not subjected to a rapid cooling treatment, was prepared. The dispersion of Reference Example 3-2 was prepared in the same manner as in Example 3, except that warming with a warmer and rapid cooling were not performed.

Test Example 2: Stability Test of Dispersion of Ultrafine Particles of Docetaxel

The dispersions of Example 3, Reference Example 3-1 and Reference Example 3-2 were charged in a glass container and then left to stand for 2 hours, respectively. After 2 hours, the solubility was visually confirmed and the laser irradiation was performed in the same manner as in Test Example 1.

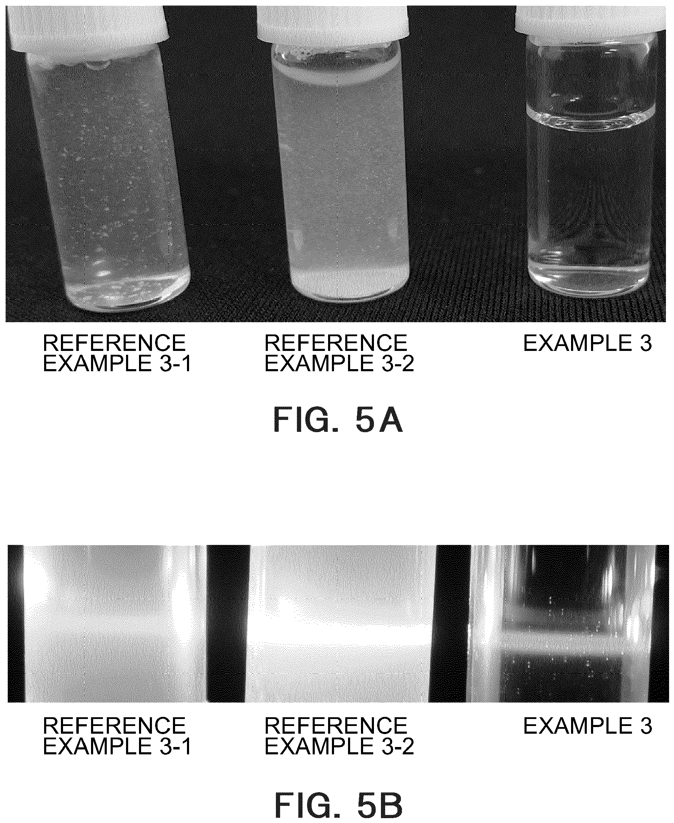

As a result of visual confirmation, the dispersion of Example 3 was colorless and transparent after being left to stand for 2 hours, and no precipitate was observed. On the other hand, the dispersions of Reference Examples 3-1 and 3-2 were suspended after being left to stand for 2 hours, and a precipitate was also observed. The results are shown in FIG. 5A.

As a result of laser irradiation, the Tyndall phenomenon of the dispersion of Example 3 was confirmed. The results are shown in FIG. 5B.

Example 4: Preparation of Dispersion of Ultrafine Particles of Paclitaxel

In a container equipped with a refluxer, 94.5 g of ethanol was charged and 5 g of EMULGEN 123P (manufactured by Kao Corporation) was added as a surfactant, followed by stirring. Thereafter, 0.5 g of paclitaxel (manufactured by Tokyo Chemical Industry Co., Ltd.) was added, followed by stirring to prepare a raw material liquid. The solubility of paclitaxel in ethanol at 25°C was 30 mg/mL or more.

The raw material liquid thus prepared as mentioned above was warmed to 50°C and then allowed to flow to a warmer with a liquid feed pump. The temperature at the outlet of the warmer of the warmed raw material liquid was 130°C

Next, the warmed raw material liquid was rapidly cooled using a micro heat exchanger. The temperature lowering rate determined from the flow rate, the equivalent diameter of the flow path, the length of the flow path, the temperature difference before and after cooling, and the like was 1,400°C/sec. The flow rate in the micro heat exchanger was 20 mL/minute. The temperature of the liquid determined by rapid cooling at the outlet of the micro heat exchanger was -10°C. The pressure in the piping of the micro heat exchanger was adjusted so as not to exceed 2.0 MPa. The liquid leaving the micro heat exchanger was a colorless transparent liquid.

To 100 mL of the liquid thus obtained, 100 mL of water was added and ethanol was distilled off by concentration under reduced pressure to obtain a water dispersion of ultrafine particles of paclitaxel. The water dispersion thus obtained was colorless transparent. The solubility of paclitaxel in water at 25°C was 0.01 mg/mL or less. The solubility was measured in the same manner as in Example 1, except that paclitaxel was used in place of coenzyme Q10.

The average particle size of the ultrafine particles of paclitaxel was measured in the same manner as in Example 1 using a transmission electron microscope. As a result, the average particle size was 3 nm. The standard deviation was 0.2605 nm.

Example 4-1 Preparation of Powder containing Ultrafine Particles of Paclitaxel

After adding an excipient obtained in Example 4, water was removed to obtain a powder containing ultrafine particles of paclitaxel. Specifically, 10 g of mannitol was added to 1 g of the water dispersion, and then the pressure was reduced to 10 Torr using a rotary evaporator R-200 and the water dispersion was vacuum-dried at 40°C. As a result, a powder containing ultrafine particles of paclitaxel was obtained.

Example 5: Preparation of Dispersion of Ultrafine Particles of Azadirachtin

In a container equipped with a refluxer, 94.5 g of ethanol was charged and 5 g of NEOCOL YSK (manufactured by Dai-ichi Kogyo Seiyaku Co., Ltd.) was added as a surfactant, followed by stirring. Thereafter, 0.5 g of azadirachtin (manufactured by FORTUNE BIO TECH LTD.) was added, followed by stirring to prepare a raw material liquid. The solubility of azadirachtin in ethanol at 25°C was 30 mg/mL or more.

The raw material liquid thus prepared as mentioned above was warmed to 50°C and then allowed to flow to a warmer with a liquid feed pump. The temperature at the outlet of the warmer of the warmed raw material liquid was 130°C

Next, the warmed raw material liquid was rapidly cooled using a micro heat exchanger. The temperature lowering rate determined from the flow rate, the equivalent diameter of the flow path, the length of the flow path, the temperature difference before and after cooling, and the like was 1,400°C/sec. The temperature of the liquid determined by rapid cooling at the outlet of the micro heat exchanger was -10°C. The flow rate in the micro heat exchanger was 20 mL/minute. The pressure in the piping of the micro heat exchanger was adjusted so as not to exceed 2.0 MPa. The liquid leaving the micro heat exchanger was a colorless transparent liquid.

To 100 mL of the liquid thus obtained, 100 mL of water was added and ethanol was distilled off by concentration under reduced pressure to obtain a water dispersion of ultrafine particles of azadirachtin. The water dispersion thus obtained was colorless transparent. The solubility of azadirachtin in water at 20°C was 0.1 mg/mL or less. The solubility was measured in the same manner as in Example 1, except that azadirachtin was used in place of coenzyme Q10, the amount of water was changed to 10 mL, and the temperature was set at 20°C.

The average particle size of the ultrafine particles of azadirachtin was measured in the same manner as in Example 1 using a transmission electron microscope. As a result, the average particle size was 3 nm. The standard deviation was 0.2589 nm.

Example 5-1 Preparation of Powder containing Ultrafine Particles of Azadirachtin

After adding an excipient obtained in Example 5, water was removed to obtain a powder containing ultrafine particles of azadirachtin. Specifically, 10 g of mannitol was added to 1 g of the water dispersion, and then the pressure was reduced to 10 Torr using a rotary evaporator R-200 and the water dispersion was vacuum-dried at 40°C. As a result, a powder containing ultrafine particles of azadirachtin was obtained.

Example 6: Preparation of Dispersion of Ultrafine Particles of Ascorbic Acid

In a container equipped with a refluxer, 70.0 g of water was charged and 5 g of EMANON 3299RV (manufactured by Kao Corporation) was added as a surfactant, followed by stirring. Thereafter, 30.0 g of L(+)-ascorbic acid (manufactured by Wako Pure Chemical Industries, Ltd.) was added, followed by stirring to prepare a raw material liquid. The solubility of L(+)-ascorbic acid in water at 25°C was 30 mg/mL or more.

The raw material liquid thus prepared as mentioned above was warmed to 50°C and then allowed to flow to a warmer with a liquid feed pump. The temperature at the outlet of the warmer of the warmed raw material liquid was 130°C

Next, the warmed raw material liquid was rapidly cooled using a micro heat exchanger. The temperature lowering rate determined from the flow rate, the equivalent diameter of the flow path, the length of the flow path, the temperature difference before and after cooling, and the like was 1,400°C/sec. The flow rate in the micro heat exchanger was 20 mL/minute. The temperature of the liquid determined by rapid cooling at the outlet of the micro heat exchanger was -10°C. The pressure in the piping of the micro heat exchanger was adjusted so as not to exceed 2.0 MPa. The liquid leaving the micro heat exchanger was a colorless transparent liquid.

To 100 mL of the liquid thus obtained, 100 mL of 1-butanol was added and water was distilled off by concentration under reduced pressure to obtain a 1-butanol dispersion of ultrafine particles of ascorbic acid. The 1-butanol dispersion thus obtained was pale yellow and transparent. The solubility of L(+)-ascorbic acid in 1-butanol at 25°C was 0.1 mg/mL or less. The solubility was measured in the same manner as in Example 1, except that L(+)-ascorbic acid was used in place of coenzyme Q10 and 100 mL of water was replaced by 10 mL of 1-butanol.

The average particle size of the ultrafine particles of ascorbic acid was measured in the same manner as in Example 1 using a transmission electron microscope. As a result, the average particle size was 3 nm. The standard deviation was 0.08651 nm.

Description of Reference Numerals

1    Raw Material Liquid

2    Liquid Feed Pump

3    Warmer

4    Micro Heat Exchanger

5    Container

6    Outlet

7    Check Valve

8    Container

**Claims**

1.  A method for manufacturing a dispersion in which a substance, which is poorly soluble in a dispersion medium containing a surfactant, is dispersed in the dispersion medium with a particle size of a nano-order level, as a dispersoid, the method comprising:

    preparing a solution containing a good solvent and the poorly soluble substance and the surfactant dissolved in the good solvent;
    rapidly cooling the solution to a temperature at which the poorly soluble substance precipitates in the solution at a temperature lowering rate of 100 to 4,000°C/second to produce ultrafine particles with a particle size of a nano-order level formed of the poorly soluble substance in the good solvent; and

    (i) separating the good solvent from a mixed solution of the solution and the dispersion medium after mixing the solution and the dispersion medium, or (ii) mixing the dispersion medium to the solution after separating the good solvent from the solution.

2.  The method according to claim 1, wherein the solution temperature is lowered using a micro heat exchanger.

3.  The method according to claim 1 or 2, wherein the separation of the good solvent in (i) is performed by distilling off the good solvent from the mixed solution of the solution and the dispersion medium.

4.  The method according to claim 1 or 2, wherein the separation of the good solvent in (ii) is performed by drying the good solvent.

5.  The method according to any one of claims 1 to 4, wherein the poorly soluble substance is at least one selected from the group consisting of coenzyme Q10, docetaxel, paclitaxel, busulfan, azadirachtin and ascorbic acid.

6. The method according to any one of claims 1 to 5, wherein the dispersion medium is at least one selected from the group consisting of water, ethanol, propylene glycol and 1-butanol.

7. The method according to any one of claims 1 to 6, wherein the good solvent is at least one solvent selected from the group consisting of acetone, ethanol, 2-propanol, propylene glycol and water.

8. The method according to any one of claims 1 to 7, wherein the average particle size of the ultrafine particles is 0.5 nm or more and 300 nm or less.

9. A method for manufacturing a powder containing ultrafine particles of a poorly soluble substance from the dispersion obtained in any one of claims 1 to 8, the method comprising optionally adding an excipient to the dispersion, and removing the dispersion medium to obtain a powder containing ultrafine particles of a poorly soluble substance.

10. Ultrafine particles with a particle size of a nano-order level formed of a poorly soluble substance, wherein the average particle size of the fine particles is 0.5 nm or more and 300 nm or less, and the coefficient of variation of the particle size is 5 or less.

**FIG. 1**

20 nm

FIG. 2

FIG. 3

EP 4 019 122 A1

REFERENCE
EXAMPLE 2-1

REFERENCE
EXAMPLE 2-2

EXAMPLE 2

FIG. 4 A

REFERENCE
EXAMPLE 2-1

REFERENCE
EXAMPLE 2-2

EXAMPLE 2

FIG. 4B

REFERENCE
EXAMPLE 3-1

REFERENCE
EXAMPLE 3-2

EXAMPLE 3

FIG. 5A

REFERENCE
EXAMPLE 3-1

REFERENCE
EXAMPLE 3-2

EXAMPLE 3

FIG. 5B

<table>
<tr><td colspan="2" align="center">**INTERNATIONAL SEARCH REPORT**</td><td>International application No.<br>PCT/JP2019/040709</td></tr>
</table>

**A.  CLASSIFICATION OF SUBJECT MATTER**
Int.Cl.  B01F3/08(2006.01)i,    A61K9/10(2006.01)i,    A61K31/10(2006.01)i,
         A61K31/337(2006.01)i, B01D9/02(2006.01)i, B01J13/00(2006.01)i

According to International Patent Classification (IPC) or to both national classification and IPC

**B.  FIELDS SEARCHED**

Minimum documentation searched (classification system followed by classification symbols)
Int.Cl.  B01F3/08, A61K9/10, A61K31/10, A61K31/337, B01D9/02, B01J13/00

Documentation searched other than minimum documentation to the extent that such documents are included in the fields searched
| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
Science Direct

**C.  DOCUMENTS CONSIDERED TO BE RELEVANT**

| Category* | Citation of document, with indication, where appropriate, of the relevant passages | Relevant to claim No. |
|---|---|---|
| X | JP 2002-518318 A (RTP PHARMA INC.) 25 June 2002, claims, paragraphs [0020]-[0022], [0033]-[0034], fig. 1-2 & US 6177103 B1, claims, column 5, lines 8-21, column 7, line 56 to column 8, line 38, fig. 1-2 & WO 1999/065469 A2 & EP 1089714 A2 & CN 1312708 A | 10 |
| A | JP 2012-236192 A (KAO CORP.) 06 December 2012, claims, paragraph [0029] (Family: none) | 1-10 |

☒  Further documents are listed in the continuation of Box C.      ☐  See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 13 December 2019 (13.12.2019) | 24 December 2019 (24.12.2019) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

| International application No. |
| --- |
| PCT/JP2019/040709 |

C (Continuation).  DOCUMENTS CONSIDERED TO BE RELEVANT

| Category* | Citation of document, with indication, where appropriate, of the relevant passages | Relevant to claim No. |
| --- | --- | --- |
| A | JP 2007-7524 A (KAGAWA INDUSTRY SUPPORT FOUNDATION) 18 January 2007, claims, paragraphs [0001], [0010]-[0014] (Family: none) | 1-10 |
| A | JP 2013-39547 A (JAPAN AGENCY FOR MARINE-EARTH SCIENCE AND TECHNOLOGY) 28 February 2013, claims, paragraph [0022] & US 2014/0296356 A1, claims, paragraph [0028] & WO 2013/027465 A1 & EP 2745928 A1 | 1-10 |
| A | WO 2005/019363 A1 (SAKURANOMIYA CHEMICAL CO., LTD.) 03 March 2005 & US 2006/0292382 A1 & EP 1659159 A1 | 1-10 |
| A | JP 8-229303 A (KONICA CORP.) 10 September 1996 (Family: none) | 1-10 |
| E, X | JP 2019-177374 A (NANO CUBE JAPAN CO., LTD.) 17 October 2019, claims, paragraphs [0045]-[0061] (Family: none) | 1-10 |

Form PCT/ISA/210 (continuation of second sheet) (January 2015)